# EUROPEAN PATENT SPECIFICATION

(11) **EP 4 144 726 B1**
(45) Date of publication and mention of the grant of the patent: **02.10.2024**
(21) Application number: 21810888.4
(22) Date of filing: 16.08.2021
(51) Int. Cl.: C07D 401/04

(54) **PREPARATION METHOD FOR SYNTHESIZING S-NICOTINE FROM GLUTARIC ACID ESTER**
HERSTELLUNGSVERFAHREN ZUR SYNTHESE VON S-NIKOTIN AUS GLUTARSÄUREESTER
PROCÉDÉ DE PRÉPARATION POUR SYNTHÉTISER DE LA S-NICOTINE À PARTIR D'ESTER D'ACIDE GLUTARIQUE

(30) Priority: 10.07.2021 CN 202110781162
(43) Date of publication of application: 08.03.2023
(73) Proprietor: Shenzhen Zinwi Bio-tech Co., Ltd, Shenzhen, Guangdong 518107 (CN)
(72) Inventor: ZOU, Jun, Shenzhen, Guangdong 518107 (CN); ZOU, Yang, Shenzhen, Guangdong 518107 (CN); LIU, Meisen, Shenzhen, Guangdong 518107 (CN); LUO, Weixian, Shenzhen, Guangdong 518107 (CN)
(74) Representative: Dr. Gassner & Partner mbB
(86) International application number: PCT/CN2021/112805
(87) International publication number: WO 2023/284059

(56) References cited:
- CN-A- 104 341 390
- HUANG KUN ET AL: "A new and efficient approach to the synthesis of nicotine and anabasine analogues", JOURNAL OF HETEROCYCLIC CHEMISTRY, vol. 46, no. 6, 1 November 2009 (2009-11-01), US, pages 1252 - 1258, XP093022372, ISSN: 0022-152X, Retrieved from the Internet <URL:https://www.ncbi.nlm.nih.gov/pmc/articles/PMC2811585/pdf/nihms168667.pdf> DOI: 10.1002/jhet.233
- HUANG KUN, ORTIZ-MARCIALES MARGARITA, DE JESÃºS MELVIN, STEPANENKO VIATCHESLAV: "A new and efficient approach to the synthesis of nicotine and anabasine analogues", JOURNAL OF HETEROCYCLIC CHEMISTRY, WILEY-BLACKWELL PUBLISHING, INC., US, vol. 46, no. 6, 1 November 2009 (2009-11-01), US , pages 1252 - 1258, XP093022372, ISSN: 0022-152X, DOI: 10.1002/jhet.233
- FELPIN, F. X. ET AL.: "Efficient Enantiomeric Synthesis of Pyrrolidine and Piperidine Alkaloids from Tobacco", THE JOURNAL OF ORGANIC CHEMISTRY, vol. 66, no. 19, 23 August 2001 (2001-08-23), pages 6305 - 6312, XP002647122, DOI: 10.1021/jo010386b
- GUARNA, A. ET AL.: "Baker's Yeast Reduction of Prochiral gamma-Nitroketones: Enantioselective Synthesis of (S)-4-Nitroalcohols", TETRAHEDRON, vol. 51, no. 6, 31 December 1995 (1995-12-31), pages 1775 - 1788, XP004104864, DOI: 10.1016/0040-4020(94)01056-6
- YE JIAN-LIANG, ZHANG YU-FENG, LIU YANG, ZHANG JIN-YUAN, RUAN YUAN-PING, HUANG PEI-QIANG: "Studies on the asymmetric synthesis of pandamarilactonines: an unexpected syn-selective vinylogous Mannich reaction of N-tert-butanesulfinimines", ORGANIC CHEMISTRY FRONTIERS, vol. 2, no. 6, 1 January 2015 (2015-01-01), pages 697 - 704, XP093022373, DOI: 10.1039/C5QO00098J

## Description

### TECHNICAL FIELD

The present invention relates to the technical field of chemical synthesis, and particularly relates to a preparation method for synthesizing S-nicotine from glutarate.

### BACKGROUND ART

Nicotine, as one of the important active ingredients of e-cigarette, is mainly extracted from tobaccos or artificially synthesized by chemical methods. Nicotine extracted and purified from plants such as tobaccos also contains other carcinogenic tobacco compound impurities that are harmful to human health; and furthermore, tobacco extracts are affected by raw materials and climate, so that it is difficult to industrially produce on a large scale. Nicotine artificially synthesized by chemical methods is almost free of other carcinogenic tobacco compound impurities, and can be industrially produced on a large scale.

A method for preparing nicotine from 3-bromopyridine is reported in Journal of Heterocyclic Chemistry, 2009, 46(6), 1252-1258., as shown in Reaction Formula 1:

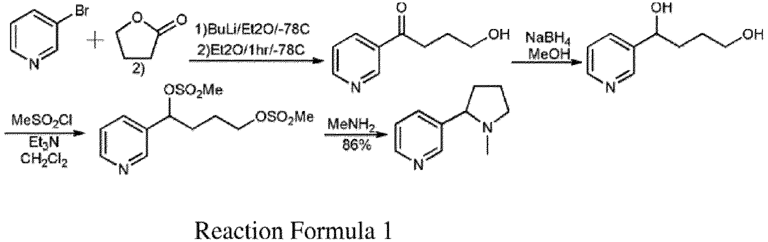

According to the preparation method of Reaction Formula 1, 3-bromopyridine is used as a starting material, which is expensive and requires an ultra-low temperature (-78°C) condition, and the experimental conditions are harsh, so that the preparation method is not suitable for industrial production, and produced nicotine is racemic nicotine.

At present, there are few preparation methods of nicotine in a single configuration with optical activity. A patent with a publication No. CN104341390A discloses a preparation method of S-nicotine. According to the method, cyclic imine is used as a starting material, an expensive chiral catalyst is required, high-pressure hydrogen equipment is required, and the production cost is relatively high, so that the method is not suitable for large-scale industrial production. HUANG KUN ET AL: "A new and efficient approach to the synthesis of nicotineand anabasine analogues" discloses that a straightforward and practical approach was established for the synthesis of nicotine and anabasine analogues by the cyclization of mesylated 1-(3-pyridinyl)-1,4, and 1,5-diol derivatives to form the pyrrolidino or piperidino fragments, Nicotine analogue ($)-15 was prepared with good enantioselectivity using the developed azacyelization procedure of nonracemie (R)-1-pyridin-3-yl-butane-1.4-diol, which was obtained by the borane-mediated reduction of ketone 12 in the presenceof the spiroborate ester derived from diphenyl prolinol and ethylene glycol. A patent with a publication No. CN11233829A discloses a preparation method of nicotine with optical activity. According to the method, a chiral ligand containing nitrogen or phosphorus is used to prepare an organometallic catalyst, an imide derivative is used as a starting material to prepare S-nicotine, the preparation of the organometallic catalyst is relatively complicated, the production cost is relatively high, and the purity of S-nicotine is relatively low.

Glutarate is a widely available and inexpensive raw material, but there is no report on the industrial synthesis of S-nicotine by using glutarate as a raw material.

### SUMMARY

In order to reduce the preparation cost of S-nicotine, the present application provides a preparation method for synthesizing S-nicotine from glutarate.

In a first aspect, the present application provides a preparation method for synthesizing S-nicotine from glutarate, which is implemented by adopting the following technical solutions:
a preparation method for synthesizing S-nicotine from glutarate, including the following steps:
S1: performing a condensation reaction on nicotinate and glutarate in the presence of a base catalyst to obtain 5-carbonyl-5-(pyridin-3-yl)pentanoic acid; a molar ratio of the nicotinate to the glutarate to the base catalyst is 1: (1-1.5): (1.2-2);
S2: reacting the 5-carbonyl-5-(pyridin-3-yl)pentanoic acid with an amination reagent to obtain 5-oxo-5-(pyridin-3-yl)pentanamide;
S3: performing a Hofmann degradation reaction on the 5-oxo-5-(pyridin-3-yl) pentanamide in the presence of hypochlorite to obtain 4-amino-1-(pyridin-3-yl)butanone;
S4: adding the 4-amino-1-(pyridin-3-yl)butanone and (+)-B-diisopinocampheyl chloroborane into an organic solvent II, and reacting to obtain (S)-4-amino-1-(pyridin-3-yl)butan-1-ol; a molar ratio of the 4-amino-1-(pyridin-3-yl)butanone to the (+)-B-diisopinocampheyl chloroborane is 1: (1.2-2); the organic solvent II is tetrahydrofuran; the reaction temperature of S4 is 0°C;
S5: reacting the (S)-4-amino-1-(pyridin-3-yl)butan-1-ol with a chlorination reagent to obtain (S)-4-amino-1-(pyridin-3-yl)chloro-butane;
S6: performing a cyclization reaction on (S)-4-amino-1-(pyridin-3-yl)butyl-1-chloride in the presence of a base to obtain S-demethylnicotine; and
S7. reacting the S-demethylnicotine with an amine methylation reagent to obtain S-nicotine.

By adopting the above technical solutions, nicotinate and glutarate are inexpensive raw materials with a wide source, so that the production cost of the raw materials can be reduced; a Claisen condensation reaction is performed on the nicotinate and the glutarate in the presence of a base catalyst to obtain 5-carbonyl-5-(pyridin-3-yl)pentanoic acid, a reaction with an amination reagent is performed to obtain 5-oxo-5-(pyridin-3-yl)pentanamide, Hofmann degradation is performed to obtain 4-amino-1-(pyridin-3-yl)butanone; a carbonyl group of the 4-amino-1-(pyridin-3-yl)butanone is reduced by using (+)-B-diisopinocampheyl chloroborane to induce the production of a chiral hydroxyl group so as to obtain (S)-4-amino-1-(pyridin-3-yl)butan-1-ol, chlorination and cyclization are performed to form chiral S-demethylnicotine, and finally amine methylation is performed to obtain S-nicotine with photochemical activity. A synthetic route using glutarate as a raw material of the present application has the advantages of simple operation, readily available raw materials, high yield, high ee value of S-nicotine, milder reaction conditions, simple treatment processes in the reaction process, etc., and is more suitable for industrial production.

More preferably, the molar ratio of the nicotinate to the glutarate to the base catalyst is 1: 1.5: 2.

In the present application, at S1, the glutarate is selected from any one of diethyl glutarate, dimethyl glutarate, di-n-propyl glutarate, and di-n-pentyl glutarate; and from the perspective of reaction cost, the cost of the glutarate being diethyl glutarate or dimethyl glutarate is lower.

In the present application, at S1, the nicotinate is methyl nicotinate or ethyl nicotinate.

In the present application, at S1, the reaction temperature of the glutarate and the base catalyst is 0 to 5°C and is preferably 0°C, the reaction time is 30 min; and the reaction temperature after the nicotinate is added is 20 to 30°C and is preferably 25°C.

In the present application, the solvent used at S1 may be one or more of tetrahydrofuran, methyl tertiary butyl ether, dimethyl tetrahydrofuran, and 1,4-dioxane; and preferably, the organic solvent I is tetrahydrofuran.

In the present application, at S1, the base catalyst is selected from one or more of alkali metal alkoxide, alkaline earth metal hydride, alkaline earth metal oxide, amine, a metal salt of amine, hydroxide, carbonate, and bicarbonate.

In the present application, the alkali metal alkoxide includes, but is not limited to, any one of sodium tert-butoxide, sodium methoxide, sodium ethoxide, and potassium tert-butoxide.

In the present application, the alkaline earth metal hydride includes, but is not limited to, one or more of NaH, LiH, and KH.

In the present application, the alkaline earth metal oxide includes, but is not limited to, one or more of Na₂O, Li₂O, and K₂O.

In the present application, the amine includes, but is not limited to, triethylamine and/or diisopropylethyl amine.

In the present application, the metal salt of amine includes, but is not limited to, sodium bis(trimethylsilyl)amide and/or lithium diisopropylamide.

In the present application, the hydroxide includes, but is not limited to, one or more of sodium hydroxide, lithium hydroxide, and magnesium hydroxide.

In the present application, the carbonate includes, but is not limited to, one or more of sodium carbonate, potassium carbonate, and cesium carbonate.

In the present application, the bicarbonate includes, but is not limited to, sodium bicarbonate and/or potassium bicarbonate.

More preferably, the base catalyst is selected from any one of sodium tert-butoxide, NaH, and potassium tert-butoxide.

In the present application, a mixture containing 5-carbonyl-5-(pyridin-3-yl)pentanoic acid is obtained at S1.

In the present application, at S2, the pH of the system needs to be adjusted before the 5-carbonyl-5-(pyridin-3-yl)pentanoic acid reacts with the amination reagent, specifically, the pH of the mixture containing 5-carbonyl-5-(pyridin-3-yl)pentanoic acid obtained at S1 is adjusted to 2 to 5, and preferably the pH of the system is adjust to 4 by using hydrochloric acid.

Preferably, at S2, the amination reagent is selected from one or more of ammonium hydroxide, formamide, and acetamide; when the amination reagent is ammonium hydroxide, the price is lower, the production cost of ammonium hydroxide is lower than the production cost of formamide and acetamide, and a subsequent deformylation and deacetylation step is not required, the reaction steps are less, which is more conducive to industrial production.

Preferably, at S2, a molar ratio of the 5-carbonyl-5-(pyridin-3-yl)pentanoic acid to the ammonium hydroxide is 1: (2-4); and more preferably, the molar ratio of the 5-carbonyl-5-(pyridin-3-yl)pentanoic acid to the ammonium hydroxide is 1: 3.

In the present application, the reaction temperature of S2 is 60 to 70°C, and the reaction time is 1 to 3 h; and preferably, the reaction temperature of S2 is 65°C, and the reaction time is 2 h.

In the present application, a mixture containing 5-oxo-5-(pyridin-3-yl)pentanamide is obtained at S2.

In the present application, at S3, the hypochlorite is selected from any one of sodium hypochlorite, sodium hypobromite, and potassium hypochlorite; and preferably, at S3, the hypochlorite is sodium hypochlorite.

In the present application, at S3, a molar ratio of the 5-oxo-5-(pyridin-3-yl)pentanamide to the hypochlorite is 1: (1-2); and preferably, the molar ratio of the 5-oxo-5-(pyridin-3-yl)pentanamide to the hypochlorite is 1: 1.5.

In the present application, at S3, specifically, the mixture containing 5-oxo-5-(pyridin-3-yl)pentanamide obtained at S2 is quickly added into the hypochlorite at 0°C, a reaction is performed at 0°C for 1 h, the temperature is raised to 71°C, the reaction is continued at 71°C for 1 h, after the reaction is stopped, the reaction solution is cooled to 25°C and added with a saturated NaOH aqueous solution, extraction is performed, an organic phase is taken, dried, and subjected to rotary evaporation for removing the solvent to obtain the 4-amino-1-(pyridin-3-yl)butanone.

Preferably, the molar ratio of the 4-amino-1-(pyridin-3-yl)butanone to the (+)-B-diisopinocampheyl chloroborane is 1: 1.5.

In the present application, the reaction time of S4 is 2 h.

In the present application, a mixture containing (S)-4-amino-1-(pyridin-3-yl)butan-1-ol is obtained at S4.

Preferably, at S5, the chlorination reagent is selected from one or more of oxalyl chloride, thionyl chloride, and trichlorophosphorus; and more preferably, the chlorination reagent is oxalyl chloride.

Preferably, at S5, a molar ratio of the (S)-4-amino-1-(pyridin-3-yl)butan-1-ol to the oxalyl chloride is 1: (1-1.5); and more preferably, the molar ratio of the (S)-4-amino-1-(pyridin-3-yl)butan-1-ol to the oxalyl chloride is 1: 1.

In the present application, the reaction temperature of S5 is 0 to 10°C; and more preferably, the reaction temperature of S5 is 0°C.

In the present application, the reaction time of S5 is 20 to 40 min; and preferably, the reaction time of S5 is 30 min.

In the present application, at S5, extraction is required after the (S)-4-amino-1-(pyridin-3-yl)butan-1-ol reacts with the oxalyl chloride, and an extraction agent may be dichloromethane or ethyl acetate. After the extraction, an organic phase is taken and subjected to rotary evaporation for removing the solvent to obtain the (S)-4-amino-1-(pyridin-3-yl)chloro-butane.

In the present application, at S6, the (S)-4-amino-1-(pyridin-3-yl)chloro-butane prepared at S5 needs to be dissolved by adding tetrahydrofuran, and after the dissolution, a reaction with a base for cyclization is performed to form the S-demethylnicotine. Preferably, at S6, the base is selected from one or more of sodium hydroxide, potassium hydroxide, lithium hydroxide, cesium hydroxide, barium hydroxide, and magnesium hydroxide; and more preferably, the base is sodium hydroxide.

In the present application, at S6, a molar ratio of the (S)-4-amino-1-(pyridin-3-yl)butyl-1-chloride to the sodium hydroxide is 1: (1.5-2.5); and preferably, the molar ratio of the (S)-4-amino-1-(pyridin-3-yl)butyl-1-chloride to the sodium hydroxide is 1: 2.

In the present application, at S6, the reaction temperature of the (S)-4-amino-1-(pyridin-3-yl)butyl-1-chloride and the base is 55 to 65°C, and the reaction time is 2 to 3 h; and preferably, the reaction temperature of the (S)-4-amino-1-(pyridin-3-yl)butyl-1 -chloride and the base is 60°C, and the reaction time is 2 h.

In the present application, a mixture containing S-demethylnicotine is obtained at S6.

In the present application, at S7, the amine methylation reagent is methyl iodide.

In the present application, at S7, a molar ratio of S-demethylnicotine in the mixture containing S-demethylnicotine to the methyl iodide is 1: (1.1-1.4); and preferably, the molar ratio of the S-demethylnicotine in the mixture containing S-demethylnicotine to the methyl iodide is 1: 1.2.

In the present application, at S7, the reaction temperature of the mixture containing S-demethylnicotine and the amine methylation reagent is 20 to 30°C, and the reaction time is 2 to 4 h; and preferably, the reaction temperature of the mixture containing S-demethylnicotine and the amine methylation reagent is 25°C, and the reaction time is 3 h.

In the present application, at S7, after the mixture containing S-demethylnicotine reacts with the amine methylation reagent, the pH needs to be adjusted to 6 by using an acid, extraction is performed, organic phases from four extractions are combined, dried over Na2SO4, and concentrated under reduced pressure to obtain crude S-nicotine, and the crude S-nicotine is purified by distillation to obtain the S-nicotine.

In summary, the present application has the following beneficial effects:

the present application provides a novel method for synthesizing S-nicotine by using cheap nicotinate and glutarate as starting materials, the raw materials are cheap, the cost is low, treatment processes in the reaction process are simple, the operation is easy, the reaction conditions are mild, the yield of S-nicotine is high, the ee value is high, and the reaction route is suitable for large-scale industrial production.

### DETAILED DESCRIPTION

The present application will be described in detail below in conjunction with embodiments.

The raw materials used in the present application can be obtained commercially, and if there is no special description, the raw materials not mentioned in the present application are purchased from Sinopharm Chemical Reagent Co., Ltd.

Embodiments 1 to 19 provide a preparation method for synthesizing S-nicotine from glutarate, which will be described below by taking Embodiment 1 as an example.

Embodiment 1 provides a preparation method for synthesizing S-nicotine from glutarate, wherein nicotinate is methyl nicotinate, the glutarate is diethyl glutarate, and a synthetic route is shown as Reaction Formula 2:

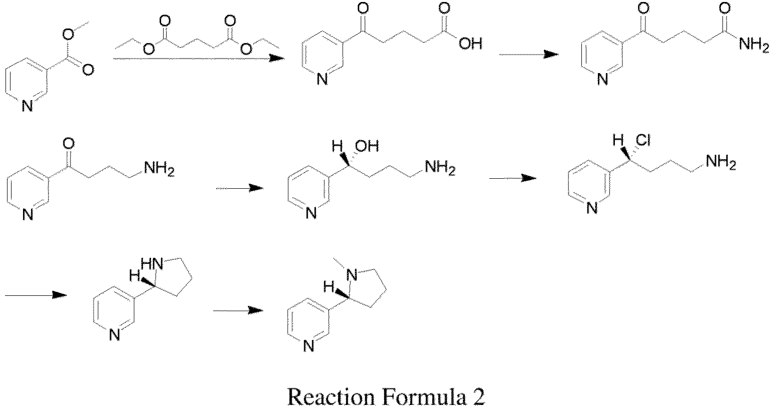

Specific preparation steps were as follows:
S1: 48 g (2 mol, 2 eq) of NaH and 282.3 g (1.5 mol, 1.5 eq) of diethyl glutarate were dissolved in 4 L of tetrahydrofuran at 0°C, a reaction was performed at 0°C for 30 min, 137.1 g (1 mol) of methyl nicotinate was added, and a condensation reaction was performed at 25°C and monitored by TCL until the end of the reaction to obtain a mixture containing 5-carbonyl-5-(pyridin-3-yl)pentanoic acid.
S2: a pH value of the mixture containing 5-carbonyl-5-(pyridin-3-yl)pentanoic acid prepared at S1 was adjusted to 4 by using 5 mol/L hydrochloric acid, and 204.36 g of industrial ammonium hydroxide (NH3: 3 mol, 3 eq) with an NH3 content of 25 wt% was added, and a reaction was performed at 65°C for 2 h to obtain a mixture containing 5-oxo-5-(pyridin-3-yl)pentanamide.
S3: the mixture containing 5-oxo-5-(pyridin-3-yl)pentanamide obtained at S2 was quickly added into 111.7 g (1.5 mol, 1.5 eq) of sodium hypochlorite at 0°C, a reaction was performed at 0°C for 1h, the water bath was heated to 71°C, the reaction was continued at 71°C for 1h; after the reaction was stopped, the reaction solution was cooled to 25°C, the pH of the system was adjusted to 9 by using a saturated NaOH aqueous solution, after the solution turned black, ethyl acetate-water (a volume ratio of the ethyl acetate to the water was 1: 2) was added for three extractions, and an organic layer was taken, dried over absolute Na₂SO₄, filtered for removing the Na₂SO₄, subjected to rotary evaporation for removing the solvent, and dried under vacuum to obtain 4-amino-1-(pyridin-3-yl)butanone.
S4: the 4-amino-1-(pyridin-3-yl)butanone obtained at S3 was dissolved in 5 L of tetrahydrofuran at 0°C, 481.1 g (1.5 mol, 1.5 eq) of (+)-B-diisopinocampheyl chloroborane was added, and a reaction was performed at 0°C for 2 h to obtain a mixture containing (S)-4-amino-1-(pyridin-3-yl)butan-1-ol;
S5: 126.9 g (1 mol, 1 eq) of oxalyl chloride was added into the mixture containing (S)-4-amino-1-(pyridin-3-yl)butan-1-ol obtained at S4 at 0°C, a reaction was performed at 0°C for 30 min, after the reaction, extraction was performed by using dichloromethane, and an organic phase was taken and subjected to rotary evaporation for removing the solvent to obtain (S)-4-amino-1-(pyridin-3-yl)chloro-butane.
S6: 2 L of tetrahydrofuran was added into the (S)-4-amino-1-(pyridin-3-yl) chloro-butane obtained at S5, after the dissolution, 80 g (2 mol, 2 eq) of NaOH was added, and after the dissolution by stirring, a reaction was performed at 60°C for 2h to obtain a mixture containing S-demethylnicotine.
S7: 170.3 g (1.2 mol, 1.2 eq) of methyl iodide was added into the mixture containing S-demethylnicotine prepared at S6, a reaction was performed at 25°C for 3 h, the pH of the system was adjusted to 6 by using 5 mol/L hydrochloric acid, extraction was performed by using dichloromethane, an organic phase was taken, added with Na₂SO₄ for drying, and concentrated under reduced pressure for removing the solvent to obtain crude S-nicotine; and the crude S-nicotine was further purified once by atmospheric distillation to obtain S-nicotine with a yield of 53%, an ee value of 98%, and a purity of 95%.

It is worthwhile to note that each mass and specific molar weight in the embodiments of the present application can be selected according to the size of an industrially produced vessel as long as the equivalence ratio of each reaction raw material is consistent.

A difference between Embodiments 2 to 3 and Embodiment 1 is that: in the reaction of S1, the kind of the base catalyst was adjusted as specifically shown in Table 1.

**Table 1 Effect of selection of base catalyst on the reaction of S 1**

| Serial number | Selection of base catalyst | Yield of S-nicotine (%) |
|---|---|---|
| Embodiment 1 | NaH | 53 |
| Embodiment 2 | Sodium tert-butoxide | 50 |
| Embodiment 3 | Potassium tert-butoxide | 50 |

A difference between Embodiments 4 to 5 and Embodiment 1 is that: in the reaction of S1, the usage amounts of the diethyl glutarate and the NaH were adjusted as specifically shown in Table 2.

**Table 2 Effect of usage amounts of diethyl glutarate and NaH on the reaction of S 1**

| Serial number | Equivalent quantity (eq) of diethyl glutarate (eq) | Amount of substance of NaH (mol) | Yield of S-nicotine (%) |
|---|---|---|---|
| Embodiment 1 | 1.5 | 2 | 53 |
| Embodiment 4 | 1.5 | 1.2 | 45 |
| Embodiment 5 | 1 | 1.2 | 48 |

A difference between Embodiments 6 to 7 and Embodiment 1 is that: in the reaction of S2, the usage amount of the ammonium hydroxide was adjusted as specifically shown in Table 3.

**Table 3 Effect of usage amount of ammonium hydroxide on the reaction of S2**

| Serial number | Equivalent quantity (eq) of ammonium hydroxide | Yield of S-nicotine (%) |
|---|---|---|
| Embodiment 1 | 3 | 53 |
| Embodiment 6 | 2 | 45 |
| Embodiment 7 | 4 | 48 |

A difference between Embodiments 8 to 9 and Embodiment 1 is that: in the reaction of S4, the usage amount of the (+)-B-diisopinocampheyl chloroborane was adjusted as specifically shown in Table 4.

**Table 4 Effect of usage amount of (+)-B-diisopinocampheyl chloroborane on the reaction of S4**

| Serial number | Equivalent quantity (eq) of (+)-B-diisopinocampheyl chloroborane | Yield of S-nicotine (%) |
|---|---|---|
| Embodiment 1 | 1.5 | 53 |
| Embodiment 8 | 1 | 49 |
| Embodiment 9 | 2 | 51 |

A difference between Embodiments 10 to 12 and Embodiment 1 is that: in the reaction of S4, the reaction temperature was adjusted as specifically shown in Table 5. Examples 10 to 15 are only comparative examples which are not examples according to the invention.

**Table 5 Effect of reaction temperature on the reaction of S4**

| Serial number | Reaction temperature (°C) | Yield of S-nicotine (%) |
|---|---|---|
| Embodiment 1 | 0 | 53 |
| Embodiment 10 | -10 | 45 |
| Embodiment 11 | 10 | 50 |
| Embodiment 12 | 5 | 49 |

A difference between Embodiments 13 to 15 and Embodiment 1 is that: in the reaction of S4, the kind of the organic solvent was adjusted as specifically shown in Table 6.

**Table 6 Effect of selection of organic solvent on the reaction of S4**

| Serial number | Selection of organic solvent | Yield of S-nicotine (%) |
|---|---|---|
| Embodiment 1 | Tetrahydrofuran | 53 |
| Embodiment 13 | 1,4-dioxane | 25 |
| Embodiment 14 | Methyl tertiary butyl ether | 0 |
| Embodiment 15 | Absolute ether | 0 |

A difference between Embodiments 16 to 17 and Embodiment 1 is that: in the reaction of S5, the usage amount of the oxalyl chloride was adjusted as specifically shown in Table 7.

**Table 7 Effect of usage amount of oxalyl chloride on the reaction of S5**

| Serial number | Equivalent quantity (eq) of oxalyl chloride | Yield of S-nicotine (%) |
|---|---|---|
| Embodiment 1 | 1 | 53 |
| Embodiment 16 | 1.5 | 49 |
| Embodiment 17 | 0.5 | 41 |

A difference between Embodiment 18 and Embodiment 1 is that: at S1, the methyl nicotinate was replaced with equimolar ethyl nicotinate, and prepared S-nicotine had a yield of 52%, an ee value of 98%, and a purity of 95%.

A difference between Embodiment 19 and Embodiment 1 is that: at S1, the diethyl glutarate was replaced with equimolar dimethyl glutarate, and prepared S-nicotine had a yield of 54%, an ee value of 98%, and a purity of 95%.

The specific embodiments are merely an explanation of the present application and are not intended to limit the present application. After reading the present description, those skilled in the art can make modifications to the present embodiments as required without any inventive contribution, and these modifications shall fall within the scope of protection of the present application.

## Claims

1. A preparation method for synthesizing S-nicotine from glutarate, **characterized by** comprising the following steps:
S1: performing a condensation reaction on nicotinate and glutarate in the presence of a base catalyst to obtain 5-carbonyl-5-(pyridin-3-yl)pentanoic acid; a molar ratio of the nicotinate to the glutarate to the base catalyst is 1: (1-1.5): (1.2-2);
S2: reacting the 5-carbonyl-5-(pyridin-3-yl)pentanoic acid with an amination reagent to obtain 5-oxo-5-(pyridin-3-yl)pentanamide;
S3: performing a Hofmann degradation reaction on the 5-oxo-5-(pyridin-3-yl)pentanamide in the presence of hypochlorite to obtain 4-amino-1-(pyridin-3-yl)butanone;
S4: adding the 4-amino-1-(pyridin-3-yl)butanone and (+)-B-diisopinocampheyl chloroborane into an organic solvent, and reacting to obtain (S)-4-amino-1-(pyridin-3-yl)butan-1-ol; a molar ratio of the 4-amino-1-(pyridin-3-yl)butanone to the (+)-B-diisopinocampheyl chloroborane is 1: (1.2-2); the organic solvent II is tetrahydrofuran; the reaction temperature of S4 is 0°C;
S5: reacting the (S)-4-amino-1-(pyridin-3-yl)butan-1-ol with a chlorination reagent to obtain (S)-4-amino-1-(pyridin-3-yl)chloro-butane;
S6: performing a cyclization reaction on (S)-4-amino-1-(pyridin-3-yl)butyl-1-chloride in the presence of a base to obtain S-demethylnicotine; and
S7. reacting the S-demethylnicotine with an amine methylation reagent to obtain S-nicotine.

2. The preparation method for synthesizing S-nicotine from glutarate according to claim 1, **characterized in that** at S2, the amination reagent is selected from one or more of ammonium hydroxide, formamide, and acetamide.

3. The preparation method for synthesizing S-nicotine from glutarate according to claim 2, **characterized in that** at S2, a molar ratio of the 5-carbonyl-5-(pyridin-3-yl)pentanoic acid to the ammonium hydroxide is 1: (2-4).

4. The preparation method for synthesizing S-nicotine from glutarate according to claim 1, **characterized in that** at S3, a molar ratio of the 5-oxo-5-(pyridin-3-yl)pentanamide to the hypochlorite is 1: (1-2).

5. The preparation method for synthesizing S-nicotine from glutarate according to claim 1, **characterized in that** at S5, the chlorination reagent is selected from one or more of oxaloyl chloride, thionyl chloride, and trichlorophosphorus.

6. The preparation method for synthesizing S-nicotine from glutarate according to claim 1, **characterized in that** at S5, a molar ratio of the (S)-4-amino-1-(pyridin-3-yl)butan-1-ol to the oxaloyl chloride is 1: (1-1.5).

## Patentansprüche

1. Herstellungsverfahren zur Synthese von S-Nicotin aus Glutarat, **dadurch gekennzeichnet, dass** es die folgenden Schritte umfasst:
S1: Durchführung einer Kondensationsreaktion mit Nikotinat und Glutarat in Gegenwart eines basischen Katalysators, um 5-Carbonyl-5-(pyridin-3-yl)-pentansäure zu erhalten; ein Molverhältnis des Nikotinats zum Glutarat zum basischen Katalysator beträgt 1: (1-1,5): (1,2-2);
S2: Umsetzung der 5-Carbonyl-5-(pyridin-3-yl)-pentansäure mit einem Aminierungsreagenz, um 5-Oxo-5-(pyridin-3-yl)-pentanamid zu erhalten;
S3: Durchführung einer Hofmann-Abbaureaktion an 5-Oxo-5-(pyridin-3-yl)pentanamid in Gegenwart von Hypochlorit, um 4-Amino-1-(pyridin-3-yl)butanon zu erhalten;
S4: Zugabe von 4-Amino-1-(pyridin-3-yl)butanon und (+)-B-Diisopinocampheylchlorboran in ein organisches Lösungsmittel und Umsetzung zum Erhalt von (S)-4-Amino-1-(pyridin-3-yl)butan-1-ol; ein Molverhältnis des 4-Amino-1-(pyridin-3-yl)butanons zum (+)-B-Diisopinocampheylchlorboran beträgt 1: (1.2-2); das organische Lösungsmittel II ist Tetrahydrofuran; die Reaktionstemperatur von S4 beträgt 0°C;
S5: Umsetzung des (S)-4-Amino-1-(pyridin-3-yl)butan-1-ols mit einem Chlorierungsreagenz, um (S)-4-Amino-1-(pyridin-3-yl)chloro-butan zu erhalten;
S6: Durchführung einer Cyclisierungsreaktion an (S)-4-Amino-1-(pyridin-3-yl)butyl-1-chlorid in Gegenwart einer Base, um S-Demethylnicotin zu erhalten; und
S7. Umsetzung des S-Demethylnicotins mit einem Amin-Methylierungsreagenz, um S-Nicotin zu erhalten.

2. Herstellungsverfahren zur Synthese von S-Nikotin aus Glutarat nach Anspruch 1, **dadurch gekennzeichnet, dass** bei S2 das Aminierungsreagenz aus einem oder mehreren von Ammoniumhydroxid, Formamid und Acetamid ausgewählt ist.

3. Verfahren zur Herstellung von S-Nicotin aus Glutarat nach Anspruch 2, **dadurch gekennzeichnet, dass** bei S2 ein Molverhältnis der 5-Carbonyl-5-(pyridin-3-yl)-pentansäure zum Ammoniumhydroxid 1: (2-4) beträgt.

4. Verfahren zur Herstellung von S-Nicotin aus Glutarat nach Anspruch 1, **dadurch gekennzeichnet, dass** bei S3 ein Molverhältnis des 5-Oxo-5-(pyridin-3-yl)pentanamids zum Hypochlorit 1: (1-2) beträgt.

5. Herstellungsverfahren zur Synthese von S-Nikotin aus Glutarat nach Anspruch 1, **dadurch gekennzeichnet, dass** bei S5 das Chlorierungsreagenz aus einem oder mehreren von Oxaloylchlorid, Thionylchlorid und Trichlorphosphor ausgewählt ist.

6. Herstellungsverfahren zur Synthese von S-Nicotin aus Glutarat nach Anspruch 1, **dadurch gekennzeichnet, dass** bei S5 ein Molverhältnis des (S)-4-Amino-1-(pyridin-3-yl)butan-1-ols zum Oxaloylchlorid 1: (1-1,5) beträgt.

## Revendications

1. Procédé de préparation pour la synthèse de S-nicotine à partir de glutarate, **caractérisé par** le fait de comprendre les étapes suivantes :
S1 : réaliser une réaction de condensation sur du nicotinate et glutarate en présence d'un catalyseur basique pour obtenir de l'acide 5-carbonyl-5-(pyridin-3-yl)pentanoïque ; un rapport molaire du nicotinate sur le glutarate sur le catalyseur basique est de 1:(1 à 1,5):(1,2 à 2) ;
S2 : faire réagir l'acide 5-carbonyl-5-(pyridin-3-yl)pentanoïque avec un réactif d'amination pour obtenir du 5-oxo-5-(pyridin-3-yl)pentanamide ;
S3 : réaliser une réaction de dégradation d'Hofmann sur le 5-oxo-5-(pyridin-3-yl)pentanamide en présence d'hypochlorite pour obtenir de la 4-amino-1-(pyridin-3-yl)butanone ;
S4 : ajouter la 4-amino-1-(pyridin-3-yl)butanone et du chloroborane de (+)-B-diisopinocamphéyle dans un solvant organique et faire réagir pour obtenir du (S)-4-amino-1-(pyridin-3-yl)butan-1-ol ; un rapport molaire de la 4-amino-1-(pyridin-3-yl)butanone sur le chloroborane de (+)-B-diisopinocamphéyle est de 1:(1,2 à 2) ; le solvant organique II est du tétrahydrofurane ; la température réactionnelle de S4 est de 0 °C ;
S5 : faire réagir le (S)-4-amino-1-(pyridin-3-yl)butan-1-ol avec un réactif de chloration pour obtenir du (S)-4-amino-1-(pyridin-3-yl)chloro-butane ;
S6 : réaliser une réaction de cyclisation sur le (S)-4-amino-1-(pyridin-3-yl)butyl-1-chlorure en présence d'une base pour obtenir de la S-déméthylnicotine ; et
S7 : faire réagir la S-déméthylnicotine avec un réactif de méthylation d'amine pour obtenir de la S-nicotine.

2. Procédé de préparation pour la synthèse de S-nicotine à partir de glutarate selon la revendication 1, **caractérisé en ce qu'**à S2, le réactif d'amination est sélectionné parmi un ou plusieurs de l'hydroxyde d'ammonium, du formamide et de l'acétamide.

3. Procédé de préparation pour la synthèse de S-nicotine à partir de glutarate selon la revendication 2, **caractérisé en ce qu'**à S2, un rapport molaire de l'acide 5-carbonyl-5-(pyridin-3-yl)pentanoïque sur l'hydroxyde d'ammonium est de 1:(2 à 4).

4. Procédé de préparation pour la synthèse de S-nicotine à partir de glutarate selon la revendication 1, **caractérisé en ce qu'**à S3, un rapport molaire du 5-oxo-5-(pyridin-3-yl)pentanamide sur l'hypochlorite est de 1:(1 à 2) .

5. Procédé de préparation pour la synthèse de S-nicotine à partir de glutarate selon la revendication 1, **caractérisé en ce qu'**à S5, le réactif de chloration est sélectionné parmi un ou plusieurs du chlorure d'oxaloyle, du chlorure de thionyle et du trichlorophosphore.

6. Procédé de préparation pour la synthèse de S-nicotine à partir de glutarate selon la revendication 1, **caractérisé en ce qu'**à S5, un rapport molaire du (S)-4-amino-1-(pyridin-3-yl)butan-1-ol sur le chlorure d'oxaloyle est de 1: (1 à 1,5).
